# EUROPEAN PATENT APPLICATION

(11) **EP 4 582 433 A1**
(43) Date of publication of application: **09.07.2025**
(21) Application number: 25150236.5
(22) Date of filing: 03.01.2025
(51) Int. Cl.: C07F 9/53, A61L 27/52, B33Y 70/00, C08B 37/08, C08J 3/075, C08J 3/28, C08L 5/08, C09D 11/101

(54) **PHOTOINTIATOR, BIOINK INCLUDING THE SAME, AND METHODE OF MANUFACTURING HYDROGEL**

(30) Priority: 05.01.2024 KR 20240002005
(71) Applicant: Pusan National University Industry - University Cooperation Foundation, Busan 46241 (KR)
(72) Inventor: YANG, Swung Yun, 50463 Miryang-si (KR); LEE, Gyeong Won, 50463 Miryang-si (KR); CHANDRASEKHARAN, Ajeesh, 50463 Miryang-si (KR); JOSE, Jeesmon, 50463 Miryang-si (KR)
(74) Representative: Mewburn Ellis LLP

(57) **Abstract**

The present disclosure relates to a photoinitiator, a bioink including the same, and a method of manufacturing a hydrogel. According to the present disclosure, the photoinitiator of the present disclosure may have water solubility, high molar extinction coefficient, and low cytotoxicity. In addition, the method of manufacturing a hydrogel of the present disclosure may have high cell viability by increasing 3D printing efficiency due to a fast photo-curing rate.

## Description

### CROSS-REFERENCE TO RELATED APPLICATION

This application claims the benefit of Korean Patent Application No. 10-2024-0002005 filed on 2024-01-05, in the Korean Intellectual Property Office, the entire disclosure of which is incorporated herein by reference for all purposes.

### BACKGROUND

### 1. Field of the Invention

The present disclosure relates to a photoinitiator, a bioink including the same, and a method of manufacturing a hydrogel.

### 2. Description of the Related Art

3D bioprinting is widely recognized as an innovative method for precisely replicating the structural complexity of native corneas. It enables the production patient-specific corneal structures. Compared to other tissues, the cornea is completely avascular requires minimal metabolism, and has a relatively uniform cell composition, making it particularly suitable for 3D bioprinting. Bioinks used in 3D bioprinting must exhibit appropriate biocompatibility and optical properties. Hydrogel-based bioinks demonstrate high cytocompatibility with living cells, excellent mechanical stability after printing, and outstanding printing resolution.

3D printing involves simple processes that ensure low cost, and it allows for the production of variety products in a small quantity without restrictions on manufacturing forms. However, in to the context of 3D bioprinting for medical applications, significant limitations remain in the selection of bioink materials.

Hyaluronic acid (HA) is a natural linear polysaccharide found in various connective tissues, including the skin, umbilical cord, and vitreous. HA-based hydrogels are highly suitable as bioprinting materials due to their high cellular compatibility, biodegradability, and the presence of numerous functional groups in HA. To utilize HA-based hydrogels for bioprinting, they must be rapidly manufactured through photocrosslinking.

A photoinitiator is a critical factor influencing the photocrosslinking rate and properties of hydrogels. For application in bioprinting, such as with HA-based hydrogels, the photoinitiator must exhibit high water solubility, perform photo-crosslinking under visible light, demonstrate fast photoreactivity, and ensure superior cell compatibility.

### [Prior-Art Document]

### [Patent Document]

Korean Patent No. 10-2612626

### SUMMARY

### Problem to be Solved by the Invention

An object of the present disclosure is to provide a photoinitiator.

Another object of the present disclosure is to provide a bioink including the photoinitiator.

Still another object of the present disclosure is to provide a method of manufacturing a hydrogel.

### Means for solving the Problem

To achieve above objects, an embodiment of the present disclosure provides a photoinitiator which has a chemical structure of the following Chemical Formula 1 and is water-soluble and activated by visible light to form free radicals: 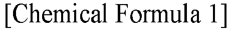 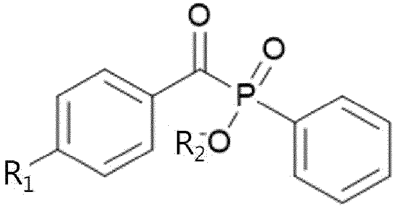 wherein, in the Chemical Formula 1, R₁ is H or F, and R₂ is Li⁺ or Mg²⁺.

In addition, an embodiment of the present disclosure provides a bioink composition including distilled water, a methacrylated hyaluronic acid (HAMA) compound dissolved in the distilled water and has a plurality of molecular weights, and a photoinitiator dissolved in the distilled water with the chemical structure of the following Chemical Formula 1: 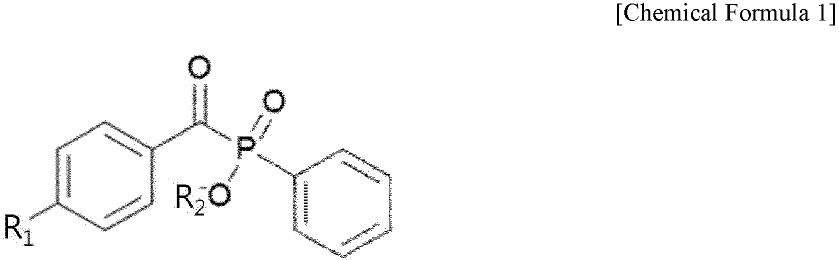 wherein, in the Chemical Formula 1, R₁ is H or F, and R₂ is Li⁺ or Mg²⁺.

In addition, an embodiment of the present disclosure provides a method for manufacturing a hydrogel, including: dissolving a methacrylated hyaluronic acid (HAMA) compound having a plurality of molecular weights and a photoinitiator with a chemical structure of the following Chemical Formula 1 in distilled water to prepare a bioink composition; printing the bioink composition while irradiating visible light to crosslink the methacrylated hyaluronic acid (HAMA) compound: 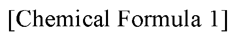 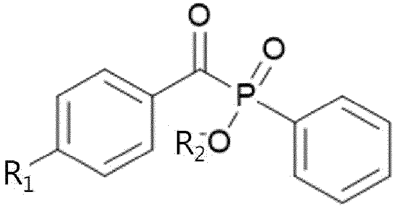 wherein, in the Chemical Formula 1, R₁ is H or F, and R₂ is Li⁺ or Mg²⁺.

### Effects of the Invention

According to the present disclosure, a photoinitiator of the present disclosure may have water solubility, high molar extinction coefficient, and low cytotoxicity. In addition, a method of manufacturing a hydrogel of the present disclosure may have high cell viability by increasing 3D printing efficiency due to the fast photo-curing rate.

### BRIEF DESCRIPTION OF THE DRAWINGS

FIG. 1 shows a flow diagram of a method of manufacturing a hydrogel according to the present disclosure.
FIG. 2 shows a schematic diagram for one Example of a method of manufacturing a corneal type hydrogel according to the present disclosure.
FIG. 3 shows a structure of a corneal type hydrogel according to the present disclosure.
FIG. 4 shows a graph evaluating solubility of photoinitiators for bioprinting according to the present disclosure in water at about 25°C.
FIG. 5 shows a graph of a molar extinction coefficient (ε)-wavelength of Examples according to the present disclosure.
FIG. 6 shows graphs of a storage coefficient and loss coefficient-time of Examples according to the present disclosure.
FIG. 7 shows a graph of cytotoxicity results of Examples according to the present disclosure.
FIG. 8 shows an image illustrating a wooden stack structure.
FIG. 9 shows images evaluating structural reproducibility in FIG. 8 of Example according to the present disclosure.
FIG. 10 shows a graph evaluating viscosity of Examples according to the present disclosure.
FIG. 11 shows a graph evaluating dispersibility of Examples according to the present disclosure.
FIG. 12 shows a cell viability-time graph of Examples according to the present disclosure.
FIG. 13 shows a graph of stress-strain curves of Examples according to the present disclosure.
FIG. 14 shows graphs illustrating tensile strength, elongation, and toughness of Examples according to the present disclosure.
FIG. 15 shows images for transparency of Examples according to the present disclosure.
FIG. 16 shows a graph of transmittance measurement of Examples according to the present disclosure.
FIG. 17 shows a shape of a corneal type hydrogel of Examples according to the present disclosure.
FIG. 18 shows results of a cell viability-time of Examples according to the present disclosure.
FIG. 19 shows images identifying characteristics of rabbit corneal stromal cells of Examples according to the present disclosure.

### DETAILED DESCRIPTION

Hereinafter, embodiments of the present disclosure will be described in detail with reference to the accompanying drawings. The present disclosure may have various modifications and forms, and thus specific embodiments will be illustrated in the drawings and described in detail in the description. However, it is not intended to limit the present disclosure to a specific form of disclosure, but should be understood to include all modifications, equivalents, or substitutes included in the spirit and technical scope of the present disclosure. In describing each drawing, similar reference numerals are used to refer to similar components. In the accompanying drawings, the enlarged dimensions are given in the constructures to ensure clarity of the present disclosure.

Terms such as first and second may be used to describe various components, but such components should not be limited by such terms. The terms are used only for distinguishing one component from another. For example, without departing from the scope of the present disclosure, the first component may be named the second component, and similarly, the second component may also be named the first component.

The terms used in this application are used only to describe particular embodiments, but not intended to limit the present disclosure. Singular expressions include plural expressions, unless the context explicitly means otherwise. As used herein, it should be understood that terms such as "comprise", "include" or "have" are intended to specify the presence of a feature, number, step, operation, component, part, or combination thereof described in the specification, but do not exclude in advance the possibility of the presence or addition of one or more other features, numbers, steps, operations, components, parts, or combinations thereof.

On the other hand, unless otherwise defined, all terms used herein, including technical or scientific terms, have the same meaning as commonly understood by those with ordinary skill in the art to which the present disclosure pertains. Terms as defined in commonly used dictionaries should be interpreted as having a meaning consistent with those in the context of the relevant art but not be interpreted in an idealized or overly formal sense, unless expressly defined otherwise in this application.

A photoinitiator according to the present disclosure has a chemical structure of the following Chemical Formula 1 and is water-soluble and activated by visible light to form free radicals: 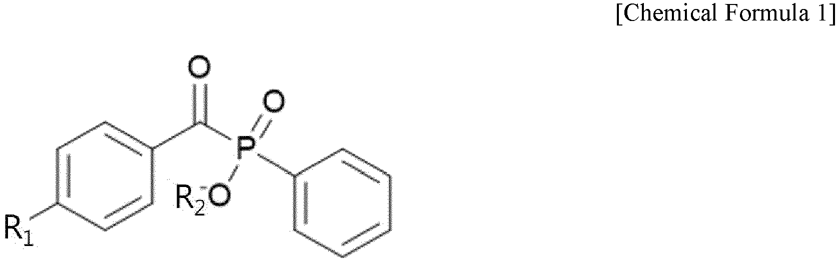 wherein, in the Chemical Formula 1, R₁ is H or F, and R₂ is Li⁺ or Mg²⁺.

In one embodiment, a solubility of the photoinitiator in water at 25°C may be 160 to 200 mM, but is not limited thereto.

In one embodiment, a molar extinction coefficient of the photoinitiator may be 40 to 80 M⁻¹cm⁻¹, but is not limited thereto.

A bioink composition according to the present disclosure may include distilled water, a methacrylated hyaluronic acid (HAMA) compound that is dissolved in the distilled water, and a photoinitiator which is dissolved in the distilled water and has a chemical structure of the following Chemical Formula 1: 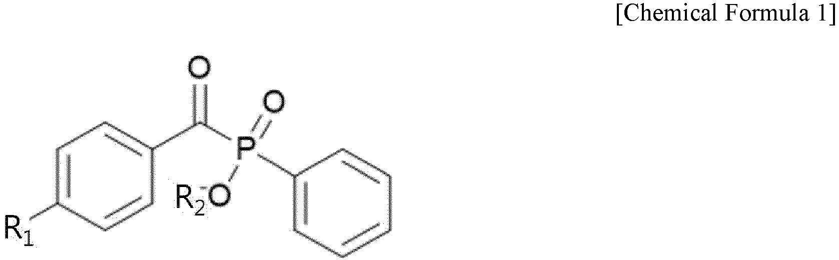 wherein, in the Chemical Formula 1, R₁ is H or F, and R₂ is Li⁺ or Mg²⁺.

In one embodiment, the methacrylated hyaluronic acid compound may have a molecular weight of about 1 to 2000 kDa.

As one embodiment, the methacrylated hyaluronic acid compound may include a compound of a single molecular weight in a range of about 1 to 2000 kDa.

As another embodiment, the methacrylated hyaluronic acid compound may include a first methacrylated hyaluronic acid compound with a molecular weight of 1 to 20 kDa and a second methacrylated hyaluronic acid compound with a molecular weight of 50 to 2000 kDa. For example, the methacrylated hyaluronic acid compound may include the first methacrylated hyaluronic acid compound with a molecular weight of about 10 kDa and the second methacrylated hyaluronic acid compound with a molecular weight of about 100 kDa.

In one embodiment, the methacrylated hyaluronic acid compound may include the first methacrylated hyaluronic acid compound and the second methacrylated hyaluronic acid compound in a molar ratio of 2 to 7:8 to 3.

In one embodiment, the bioink composition may include about 5 to 15 mass% of the methacrylated hyaluronic acid compound and about 0.02 to 5 mass% of the photoinitiator.

In one embodiment, viscosity of the bioink composition may be about 30 to 70 mPa·s, but is not limited thereto.

In one embodiment, the bioink composition may further include a photoabsorber. As an embodiment, tartrazine may be used as the photoabsorber.

In one embodiment, the bioink composition may further include a cell. As one embodiment, a corneal stromal cell may be used as the cell.

In one embodiment, the bioink composition may further include collagen.

In one embodiment, the bioink composition may be a composition for corneal formation.

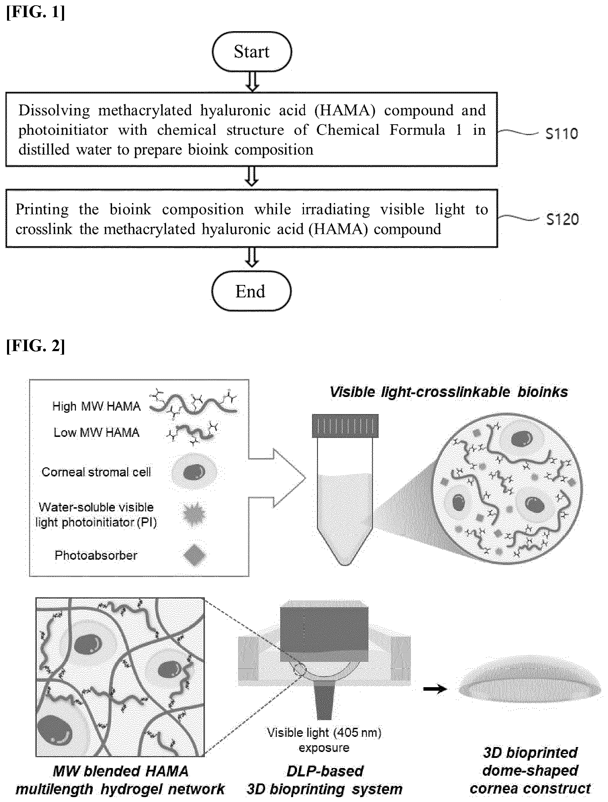

FIG. 1 shows a flow diagram of a method of manufacturing a hydrogel according to the present disclosure.

Referring to FIG. 1, the method of manufacturing a hydrogel according to the present disclosure may include dissolving a methacrylated hyaluronic acid (HAMA) compound and a photoinitiator with a chemical structure of the following Chemical Formula 1 in distilled water to prepare a bioink composition; printing the bioink composition while irradiating visible light to crosslink the methacrylated hyaluronic acid (HAMA) compound: 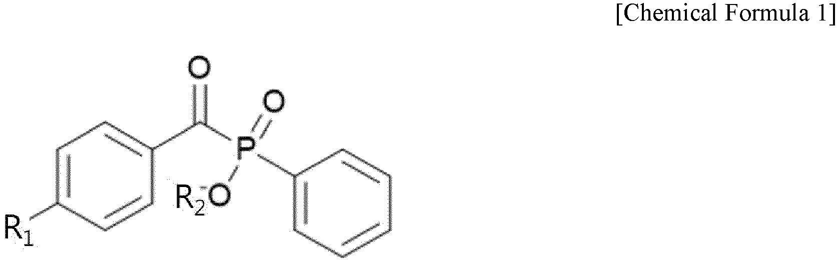 wherein, in the Chemical Formula 1, R₁ is H or F, and R₂ is Li⁺ or Mg²⁺.

In one embodiment, the methacrylated hyaluronic acid (HAMA) compound may have a single or a plurality of molecular weights within a range of about 1 to 2000 kDa.

In one embodiment, a thickness of the hydrogel may be about 300 to 700 µm. As an embodiment, the thickness of the hydrogel may be about 500 µm.

In one embodiment, the hydrogel may have a transmittance of about 70 to 98 % in light with a wavelength of about 450 to 600 nm.

In one embodiment, tensile strength of the hydrogel may be about 150 to 250 kPa, elongation may be about 20 to 55%, and toughness may be about 10 to 40 kJ/m³, but are not limited thereto.

### EXAMPLES

Hereinafter, the present disclosure will be described in more detail through examples to help understanding of the present disclosure. However, examples below are merely intended to illustrate the content of the present disclosure, and the scope of the present disclosure is not limited to the following examples. Examples of the present disclosure are provided to more completely explain the present disclosure to those skilled in the art.

### <Preparation Example 1> Synthesis of methacrylated hyaluronic acid (HAMA)

Hyaluronic acid (HA, about 0.5 g, molecular weight: about 100 kDa) was dissolved in distilled water (5 mL), and sodium hydroxide (about 1 N) was used at about 0 to 5°C to adjust the pH to 8.0 to prepare a mixture. To the mixture, 4 equivalents of anhydrous methacrylate were added dropwise for about 30 minutes based on the disaccharide unit of HA. The mixture was precipitated in ethanol followed by filtration, and the obtained solid was washed with ethanol, frozen at about -30°C, and then lyophilized to be stored at -20°C until use.

### <Preparation Example 2> Synthesis of methacrylated hyaluronic acid (HAMA)

Except that the molecular weight of hyaluronic acid in Preparation Example 1 was about 10 kDa, the HAMA was prepared using the same method as in Preparation Example 1.

### <Example 1> Preparation of a photoinitiator

Dimethyl phenylphosphonite (about 187 mg, about 1.1 mmol) was added to benzoyl chloride (about 140 mg, about 1 mmol) and 2-butanone (about 10 ml) and stirred in the presence of nitrogen for about 24 hours at room temperature to prepare a first mixture. Lithium bromide (about 95 mg, about 1.1 mmol) was added to 2-butanone (about 10 mmol) solution to prepare a second mixture. The first mixture and the second mixture were mixed, heated at about 60°C for about 20 minutes, and cooled to room temperature to produce a reactant. The reactant was washed with 2-butanone and diethyl ether in turn, and then dried under vacuum to obtain a photoinitiator for bioprinting, which was named LBP (about 237 mg, about 94% yield).

¹H NMR (600 MHz, D₂O): 8.09 (d, 2 H), 7.61-7.65 (m, 2H), 7.58 (t, 1H), 7.49 (t, 1H), 7.46-7.42 (m, 4H). ¹³C NMR (151 MHz, D₂O): 212.39, 211.61,135.72, 135.43, 134.53, 133.54, 132.66, 131.91, 131.89, 131.70, 131.64, 129.18, 129.16, 128.83, 128.46, 128.36. ³¹P NMR (243 MHz, D₂O):18.37. HRMS (ESI MS) m/z: calculated: 252.13 found:253.06 ([M+H]+ detected)

### <Example 2> Preparation of a photoinitiator

Except that 4-fluorobenzoyl chloride (about 158 mg, about 1 mmol) was used instead of the benzoyl chloride in Example 1, a photoinitiator for bioprinting was prepared using the same method as in Example 1 and named LFBP.

¹H NMR (600 MHz, D₂O): 8.18-8.17 (m, 2H), 7.64-7.61 (m, 2H), 7.50 (t, 1H), 7.44-7.42 (m, 2H), 7.17 (t, 2H). ¹³C NMR (151 MHz, D₂O): 210.53, 209.74, 167.03, 165.34, 133.41, 132.53, 132.27, 132.21, 131.99, 131.97, 131.95, 131.93, 131.71, 131.65, 128.46, 128.39. ³¹P NMR (243 MHz, D₂O):18.59. HRMS (ESI MS) m/z: calculated,270.12 found:271.05 ([M+H]+ detected)

### <Example 3> Preparation of a photoinitiator

Except that magnesium bromide (about 203 mg, about 1.1 mmol) was used instead of the lithium bromide in Example 1, a photoinitiator for bioprinting was prepared using the same method as in Example 1 and named MBP.

¹H NMR (600 MHz, D₂O): 8.14-8.13 (d, 2H), 7.70-7.65 (m, 2H), 7.65-7.60 (t, 1H), 7.44-7.42 (t, 2H), 7.51-744 (m, 4H).

### <Example 4> Bioink

Bioink was prepared by dissolving LBP (about 1.0 g), Preparation Example 1 (about 5.0 g), Preparation Example 2 (about 5.0 g), and tartrazine (about 3 mM) in about 100 ml of distilled water.

### <Example 5> Bioink

LBP (about 1.0 g), Preparation Example 1 (about 5.0 g), and Preparation Example 2 (about 5.0 g) were dissolved in about 100 ml of PBS.

### <Example 6> Hydrogel

Bioink was prepared by dissolving LBP (about 1.0 g), Preparation Example 1 (about 9.0 g), and Preparation Example 2 (about 1.0 g) in about 100 ml of distilled water. The bioink was used to prepare a hydrogel through light irradiation (about 405 nm, ~ 6.8 mW/cm², about 15 seconds) using a DLP printer (Perfactory Micro Plus HD, EnvisionTEC, Germany) in a dog-bone shape with a thickness of about 1 mm according to the ASTM D1708 test method.

### <Example 7> Hydrogel

Bioink was prepared by dissolving LBP (about 1.0 g), Preparation Example 1 (about 7.0 g), and Preparation Example 2 (about 3.0 g) in about 100 ml of distilled water. The bioink was used to prepare a hydrogel through light irradiation (about 405 nm, ~ 6.8 mW/cm², about 15 seconds) using the DLP printer (Perfactory Micro Plus HD, EnvisionTEC, Germany) in a dog-bone shape with a thickness of about 1 mm according to the ASTM D1708 test method.

### <Example 8> Hydrogel

Bioink was prepared by dissolving LBP (about 1.0 g), Preparation Example 1 (about 5.0 g), and Preparation Example 2 (about 5.0 g) in about 100 ml of distilled water. The bioink was used to prepare a hydrogel through light irradiation (about 405 nm, ~ 6.8 mW/cm², about 15 seconds) using the DLP printer (Perfactory Micro Plus HD, EnvisionTEC, Germany) in a dog-bone shape with a thickness of about 1 mm according to the ASTM D1708 test method.

### <Example 9> Hydrogel

Bioink was prepared by dissolving LBP (about 1.0 g), Preparation Example 1 (about 3.0 g), and Preparation Example 2 (about 7.0 g) in about 100 ml of distilled water. The bioink was used to prepare a hydrogel through light irradiation (about 405 nm, ~ 6.8 mW/cm², about 15 seconds) using the DLP printer (Perfactory Micro Plus HD, EnvisionTEC, Germany) in a dog-bone shape with a thickness of about 1 mm according to the ASTM D1708 test method.

### <Example 10> Hydrogel

Bioink was prepared by dissolving LBP (about 1.0 g), Preparation Example 1 (about 5.0 g), and Preparation Example 2 (about 5.0 g) in about 100 ml of distilled water. The bioink was used to prepare a hydrogel through light irradiation (about 405 nm, ~ 6.8 mW/cm², about 15 seconds) using the DLP printer (Perfactory Micro Plus HD, EnvisionTEC, Germany) with an average thickness of about 500 µm (hereinafter referred to as bHAMA).

### <Example 11> Corneal shape hydrogel

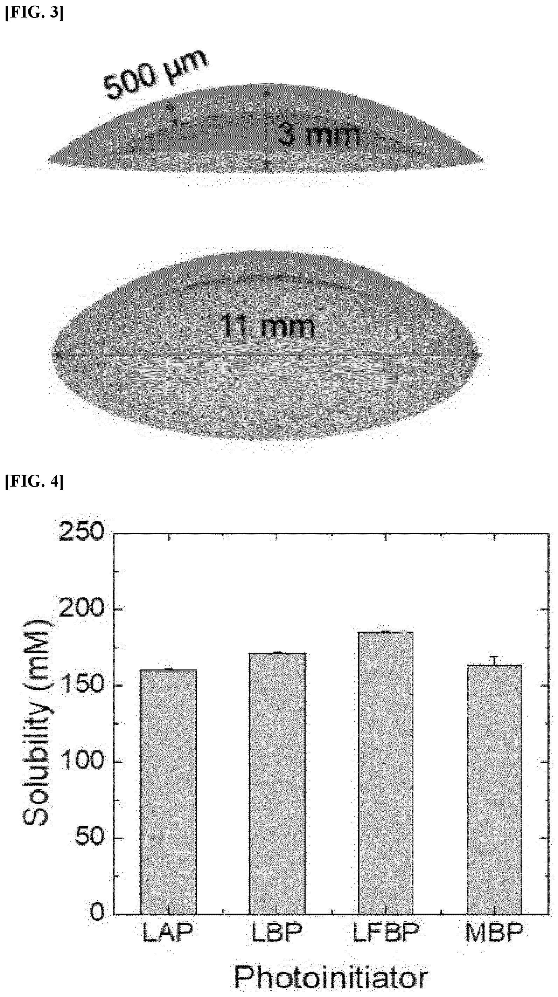

FIG. 2 shows a schematic diagram of one embodiment of a method for manufacturing a corneal shape hydrogel according to the present disclosure. FIG. 3 shows the structure of a corneal shape hydrogel according to the present disclosure. Referring to FIGS. 2 and 3, bioink was prepared by dissolving LBP (about 1.0 g), Preparation Example 1 (about 5.0 g), Preparation Example 2 (about 5.0 g), tartrazine (about 3 mM), and rabbit corneal stromal cells (1 × 10⁶ cells/mL) in about 100 ml of distilled water. The bioink was used to prepare a hydrogel, as shown in FIG. 3, through light irradiation (about 405 nm, ~ 6.8 mW/cm², about 15 seconds) using the DLP printer (Perfactory Micro Plus HD, EnvisionTEC, Germany). The hydrogel was immersed in DMEM medium which was then replaced every 20 minutes to remove the LBP and tartrazine remaining in the hydrogel.

### <Comparative Example 1> Photoinitiator for bioprinting

Lithium phenyl-2,4,6-trimethylbenzoylphosphination (LAP), a commercial photoinitiator for bioprinting, was used (hereinafter referred to as LAP).

### <Comparative Example 2> Bioink

Bioink was prepared by dissolving LBP (about 1.0 g), Preparation Example 1 (about 10.0 g), and tartrazine (about 3 mM) in about 100 ml of distilled water.

### <Comparative Example 3> Bioink

Bioink was prepared by dissolving LBP (about 1.0 g), Preparation Example 2 (about 10.0 g), and tartrazine (about 3 mM) in about 100 ml of distilled water.

### <Comparative Example 4>

LBP (about 1.0 g) and gelatin methacryloyl (GelMA, about 10 g) were dissolved in about 100 ml of PBS.

### <Comparative Example 5>

LBP (about 1.0 g) and poly (ethylene glycol) diacrylate (PEGDA, about 10 g) were dissolved in about 100 ml of PBS.

### <Comparative Example 6> Bioink

Bioink was prepared by dissolving LBP (about 1.0 g) and Preparation Example 1 (about 10.0 g) in about 100 ml of distilled water. The bioink was used to prepare a hydrogel through light irradiation (about 405 nm, ~ 6.8 mW/cm², about 15 seconds) using the DLP printer (Perfactory Micro Plus HD, EnvisionTEC, Germany) in a dog-bone shape with a thickness of about 1 mm according to the ASTM D1708 test method.

### <Comparative Example 7> Bioink

Bioink was prepared by dissolving LBP (about 1.0 g) and Preparation Example 2 (about 10.0 g) in about 100 ml of distilled water. The bioink was used to prepare a hydrogel through light irradiation (about 405 nm, ~ 6.8 mW/cm², about 15 seconds) using the DLP printer (Perfactory Micro Plus HD, EnvisionTEC, Germany) in a dog-bone shape with a thickness of about 1 mm according to the ASTM D1708 test method.

### <Comparative Example 8>

Bioink was prepared by dissolving gelatin methacryloyl (GelMA, about 10 g) in about 100 ml of distilled water. The bioink was used to prepare a hydrogel through light irradiation (about 405 nm, ~ 6.8 mW/cm², about 15 seconds) using the DLP printer (Perfactory Micro Plus HD, EnvisionTEC, Germany) in an average thickness of about 500 µm (hereinafter referred to as GelMA).

### <Comparative Example 9>

Bioink was prepared by dissolving poly (ethylene glycol) diacrylate (PEGDA, about 10 g) in about 100 ml of distilled water. The bioink was used to prepare a hydrogel through light irradiation (about 405 nm, ~ 6.8 mW/cm², about 15 seconds) using the DLP printer (Perfactory Micro Plus HD, EnvisionTEC, Germany) in an average thickness of about 500 µm (hereinafter referred to as PEGDA).

### <Experimental Example 1> Evaluation of performance of the photoinitiator

FIG. 4 shows a graph evaluating solubility of the photoinitiators for bioprinting according to the present disclosure in water at about 25°C. LBP, LFBP, and MBP exhibited maximum solubility of about 170.5 mM, 184.7 mM, and 163.2 mM, respectively, which was higher than the LAP (about 159.7 mM). Thereby, it was determined that LBP, LFBP, and MBP are soluble in water without additional solvent and heating.

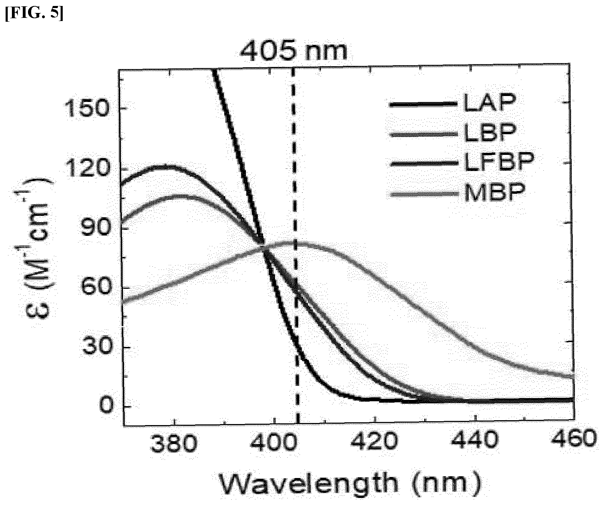

FIG. 5 is a graph of a molar extinction coefficient (ε)-wavelength of Examples according to the present disclosure. The ultraviolet-visible light absorption spectra of LBP, FLBP, MBP, and LAP were recorded by a spectrophotometer (Shimadzu UV-1800) in distilled water using about 10 mm quartz cuvettes, and the molar extinction coefficient was measured in M⁻¹·cm⁻¹. The molar extinction coefficient is an intrinsic property that affects the efficiency of the photoinitiator, and LBP (about 60 M⁻¹·cm⁻¹), LFBP (about 56 M⁻¹·cm⁻¹), and MBP (about 88 M⁻¹·cm⁻¹) showed about 2 times higher molar extinction coefficients compared to LAP (about 29 M⁻¹·cm⁻¹) at about 405 nm. LBP and LFBP showed similar absorption patterns, identifying that the substitution of hydrogen atoms with electrically negative fluorine atoms did not have a significant effect on the performance of the molar extinction coefficient. In addition, MBP, in which Li⁺ ions were substituted with highly biocompatible Mg²⁺ ions, had the best properties in terms of molar extinction coefficient.

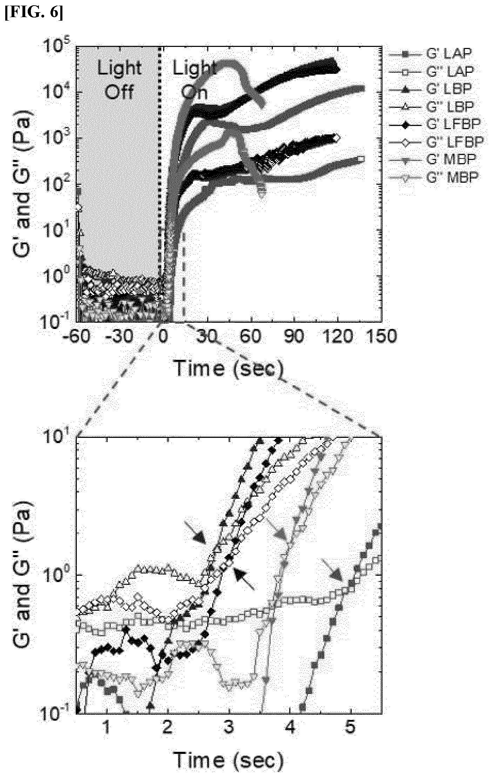

FIG. 6 shows a graph of a storage coefficient and loss coefficient-time of Examples according to the present disclosure. The rheological properties were measured to determine curing rates of LBP, LFBP, MBP, and LAP. The intersection point of the storage modulus (G') and the loss modulus (G") is at a point of gelation, and the curing rate of the LBP, LFBP, MBP, and LAP may be determined by the intersection points. The rheological properties of LBP, LFBP, MBP, and LAP were measured using a solution in which about 10 g of Preparation Example 1 and LBP, LFBP, MBP, or LAP (about 1.0 g) are dissolved in about 100 ml of distilled water. The measurement of the rheological properties was carried out using a photo-curing system (405 nm) and a stress-controlled rheometer (MCR 302, Anton Paar, Austria) equipped with a temperature-controlled water bath. The changes over time in the storage modulus (G') and loss modulus (G") of LBP, LFBP, MBP, and LAP were measured under light irradiation of about 405 nm (about 6.8 mW/cm²) and oscillation of 1 rad⁻¹. Time-sweep vibration tests were conducted with a plate-plate geometry at about 25°C, with a frequency of 1 Hz, a 0.4 mm spacing, and a 1% strain. The intersection point of G' and G" was a point of gelation, and LBP (about 2.76 s), LFBP (about 3.0 s), and MBP (about 4.0 s) showed faster curing rates than LAP (about 4.73 s). Photoinitiators with faster curing rates allow for faster printing, while retaining consistency in the shape, and shorten the exposure time of cells to the photoinitiators.

### <Experimental Example 2> Cytotoxicity of the photoinitiator

### Cell Culture

All animal testing procedures were approved by the Institutional animal care and use Committee of Pusan National University Hospital (Approval Number: PNUH-2020-162). Rabbit eyes (adult white New Zealand rabbits) were removed from euthanized animals to obtain rabbit corneal stromal cells. Rabbit corneal stromal cells were cultured in a cell culture dish with a size of about 100 mm along with DMEM supplemented with about 10% FBS and penicillin-streptomycin (about 100 UI/ml). The cultured cells were kept at about 37°C in a sterile culture machine (Labogene, Seoul, Korea) containing about 5% CO₂ until reaching the confluence.

Murine NIH-3T3 fibroblasts purchased from ATCC (Manassas, VA, USA) were cultured in a cell culture dish in a size of about 100 mm along with DMEM supplemented with about 10% FBS and penicillin-streptomycin (about 100 UI/ml). The cultured cells were kept at about 37°C in a sterile culture machine (Labogene, Seoul, Korea) containing about 5% CO₂ until reaching the confluence.

### Cytotoxicity Experiment

The cytotoxicity of LBP, MBP, and LAP was assessed by measuring cell viability after exposure to various concentrations. To conduct cell viability analysis, fibroblasts were seeded into 24-well-plates (2 × 10⁴ cells/well) and cultured in a sterile culture machine at about 37°C for about 12 hours, and then the media were removed. The LBP, MBP and LAP were diluted in media having various concentrations (0, 0.025, 0.05, 0.1, and 0.2 %(w/v)), and then the fibroblasts were cultured for about 24 hours in a sterile incubator at about 37°C, followed by washing with PBS (1X).

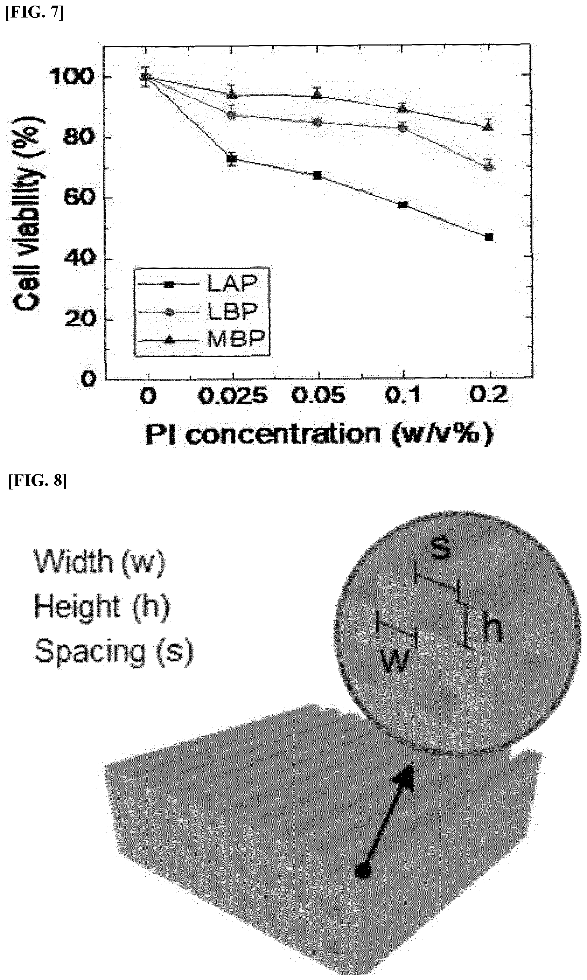

FIG. 7 shows a graph of cytotoxicity results of Examples according to the present disclosure. The cytotoxicity of LAP, LBP, and MBP for fibroblasts was compared at various concentrations ranging from 0.025 to 0.2%. At all test concentrations, LBP and MBP showed lower cytotoxicity than LAP. At a concentration of about 0.2% (w/v), the cell viability of LAP was found to be about 46%, while that of LBP and MBP was about 69% and 83%, respectively. In particular, by substituting with highly biocompatible Mg²⁺ ions, it was identified that there was a remarkable effect on reduction in the cytotoxicity.

### <Experimental Example 3> Evaluation on printability of the bioink

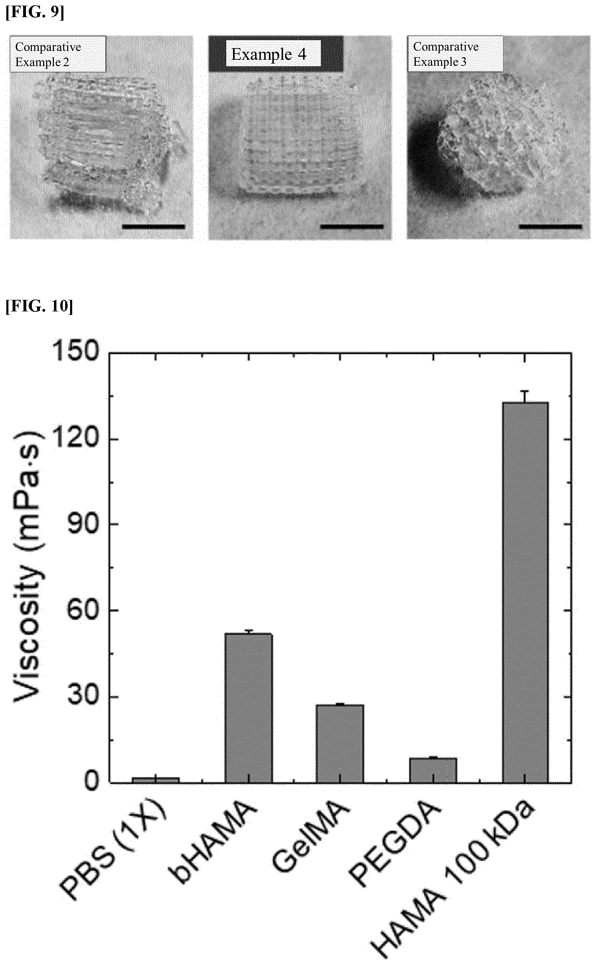

FIG. 8 shows an image illustrating a wooden stack structure. Referring to FIG. 8, the bioinks of Example 4, Comparative Example 2, and Comparative Example 3 were prepared by light irradiation (about 405 nm, ~ 6.8 mW/cm², about 15 seconds) using the DLP printer (Perfactory Micro Plus HD, EnvisionTEC, Germany). A hydrogel with a structure of FIG. 8 was prepared and shown in FIG. 9. As shown in FIG. 9, in Example 4, a wooden stack structure with distinctive check patterns was fabricated, but the structures in Comparative Example 2 and Comparative Example 3 collapsed.

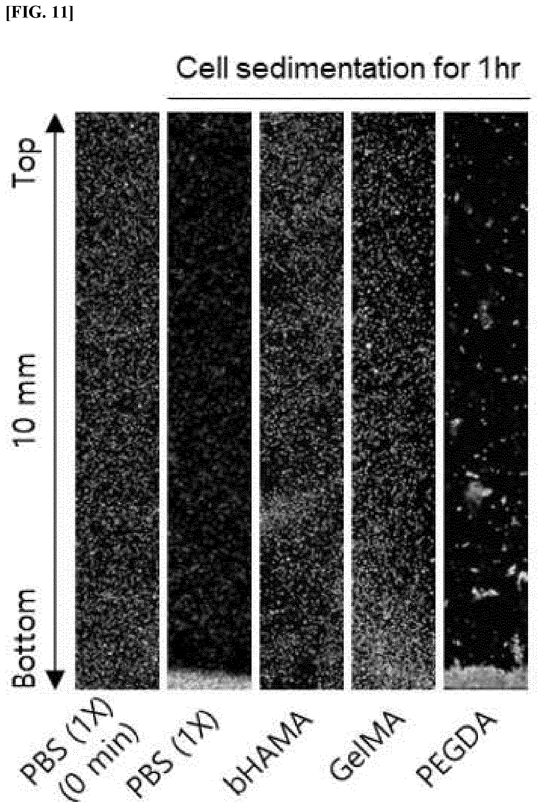

FIG. 10 shows a graph evaluating viscosity of Examples according to the present disclosure, and FIG. 11 shows a graph evaluating dispersibility of Examples according to the present disclosure. In order to evaluate the viscosity of the bioink and the dispersion stability of cells, rabbit corneal stromal cells (1 × 10⁶ cells/mL) were dispersed in Example 5, Comparative Example 4, and Comparative Example 5 (10% (w/v) in PBS (1X)), respectively. The viscosity of 10% (w/v) bioink dissolved in PBS(1X) was measured using a viscometer (µVISC, RheoSense, USA) at about 25°C. The cell-bioink solution was filled into a mold placed between two glass slides separated at intervals of about 500 µm, with a vertical setting retained. After about 1 hour, confocal microscopy (K1-Fluo, nanoscope systems, Korea) was used to evaluate the dispersion stability of the cells in the bioink. In FIG. 10, the viscosity of Example 5, Comparative Example 4, and Comparative Example 5 was found to be about 51.9 mPa·s, about 27.1 mPa·s, and about 8.6 mPa·s, respectively. Example 5 exhibited a favorably high viscosity for long-term dispersion stability. In FIG. 11, cells dispersed in Comparative Example 4 and Comparative Example 5 with a low viscosity were precipitated within 1 hour. On the other hand, in Example 5, consistent cell dispersion was observed.

### <Experiment Example 4> Cell viability of the bioink

Rabbit corneal stromal cells cultured in Experimental Example 2 were used. Rabbit corneal stromal cells were cultured in LBP (about 1% (w/v)) and washed with PBS (1X) at intervals of about 30, 60, 120, 240, and 360 minutes. To measure the cell viability, cells were supplemented with about 10 µl of WST-1 reagent (EZ-Cytox) and cultured for about 1 to 2 hours, and then cell viability was measured via a colorimetric assay at about 450 nm using a microplate reader (AMR-100, Allsheng Co., Ltd., China).

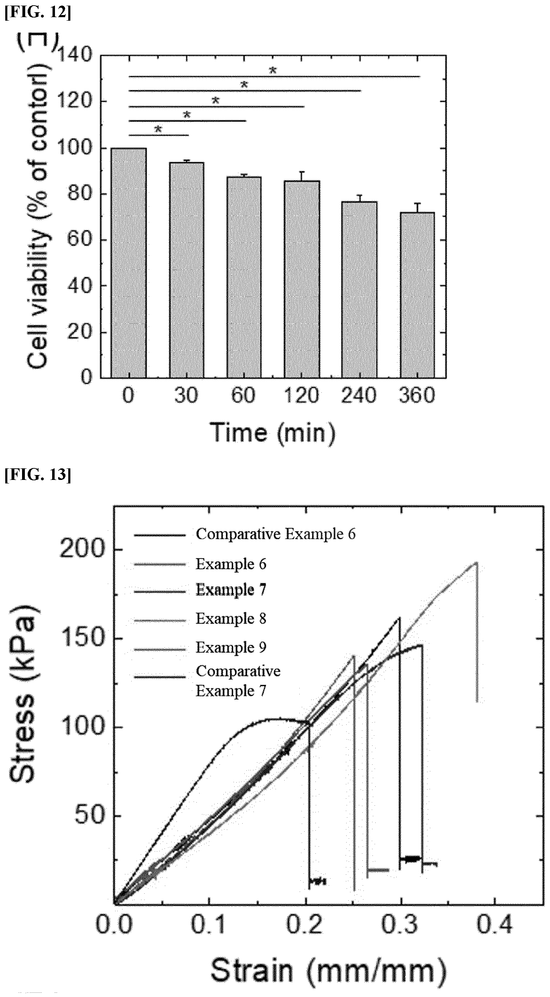

FIG. 12 shows a cell viability-time graph of Examples according to the present disclosure. As time passed, the cell viability gradually decreased, but it remained above 85% after about 1 hour. Considering the production of constructures in several centimeters within about 1 hour at a fast printing rate of the DLP printer (about 100 µm/min or more), when applying Example 4 to the industry, a cell viability that surpasses expectations will be derived. Thereby, the bioinks according to the present disclosure may be used for 3D bioprinting without adversely affecting the cell viability. Combining the high molar extinction coefficient in the visible region with the fast printing rate of LBP and DLP with low cytotoxicity, it is possible to increase cell viability by minimizing light exposure time.

### <Experiment Example 5> Mechanical properties of the hydrogel

The mechanical properties of Example 6 to Example 9, Comparative Example 6, and Comparative Example 7 were measured using a tensile tester (34SC-1, Instron, USA) at a strain of about 1 mm/min in tensile mode, which is shown in FIG. 13. Tensile strength and elongation were measured at the maximum point of stress and strain, respectively, on the stress-strain curve, and toughness was calculated from the area under the stress-strain curve to the break. The elastic modulus was assessed by obtaining an initial slope of about 5% on the strain-stress curve.

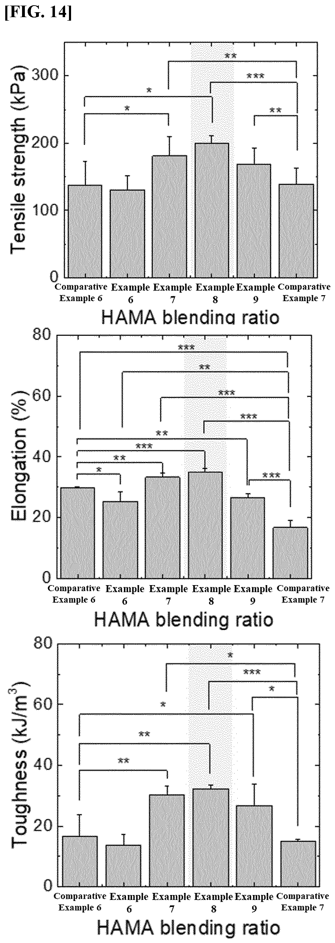

FIG. 14 shows graphs illustrating tensile strength, elongation, and toughness of Examples according to the present disclosure. In Example 7 and Example 8, tensile strength, elongation, and toughness were enhanced in comparison with Comparative Example 6 and Comparative Example 7. Example 8 had the highest records in the tensile strength (about 200.46 kPa), elongation (about 35%), and toughness (about 32.35 kJ/m³).

### <Experimental Example 5> Optical properties of the hydrogel

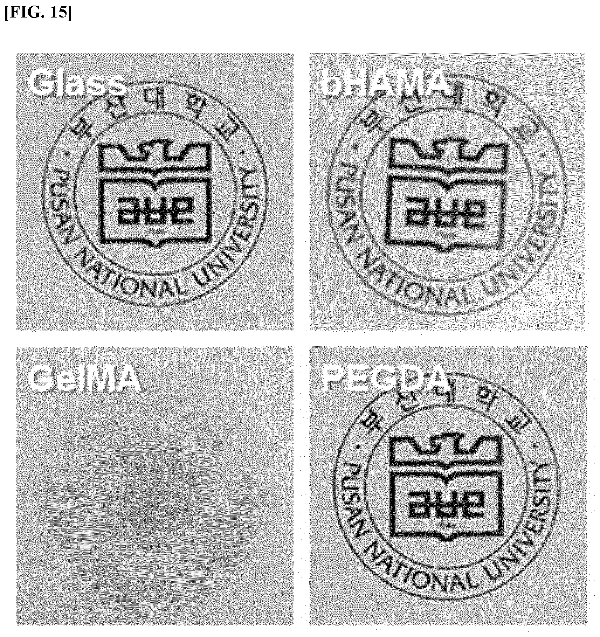

FIG. 15 shows images of transparency of Examples according to the present disclosure. Since the cornea is the transparent part of the front of the eye that transmits light, the optical transparency of the printed bioink is a crucial factor in corneal transplantation. In Example 10, Comparative Example 8, and Comparative Example 9, a hydrogel was fabricated according to the thickness of corneas (about 500 µm). When Example 10, comparative Example 8, and Comparative Example 9 were placed on the images and visually observed, Example 10 showed outstanding clarity and transparency. However, Comparative Example 8 created a blurry image due to low transparency, and Comparative Example 9 was shown in yellow.

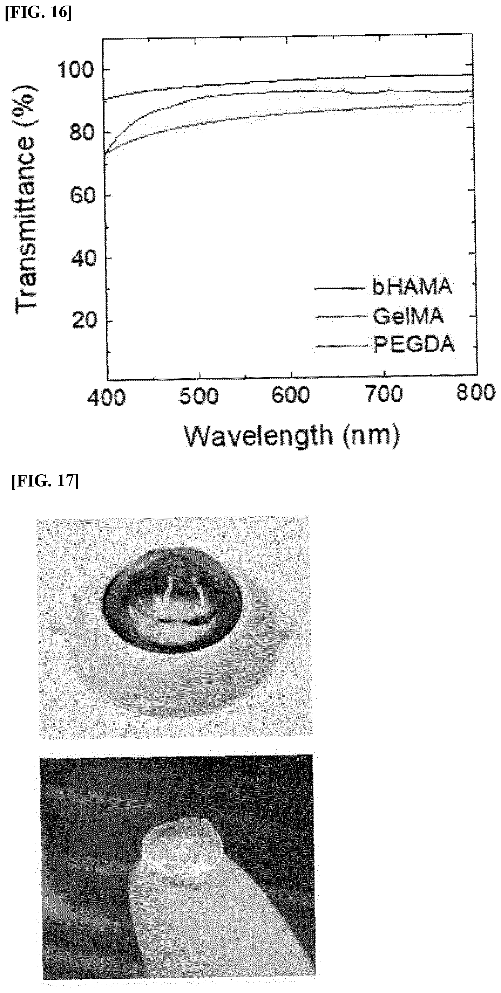

FIG. 16 shows a graph of transmittance measurement of Examples according to the present disclosure. A UV-Vis spectroscopy (Libra S70, Biochrom, UK) was used to measure the transmittance in the visible light region (400 to 800 nm). Prior to measurement, immersion was performed in PBS (1X) for about 1 hour to remove the residual LBP and tartrazine. Example 10 and Comparative Example 9 showed high transmittance of about 91 to 97% and about 72 to 91%, respectively. In the case of Comparative Example 8, a low light transmittance of about 73 to 88% was shown.

### <Experimental Example 6> Corneal type hydrogel

FIG. 17 shows a shape of the corneal type hydrogel of Examples according to the present disclosure. Referring to FIG. 17, it was found that the corneal type hydrogel with less thickness (about 500 µm) has compartmental adhesiveness, and the corneal type hydrogel maintains its structure even after removal of LBP and tartrazine.

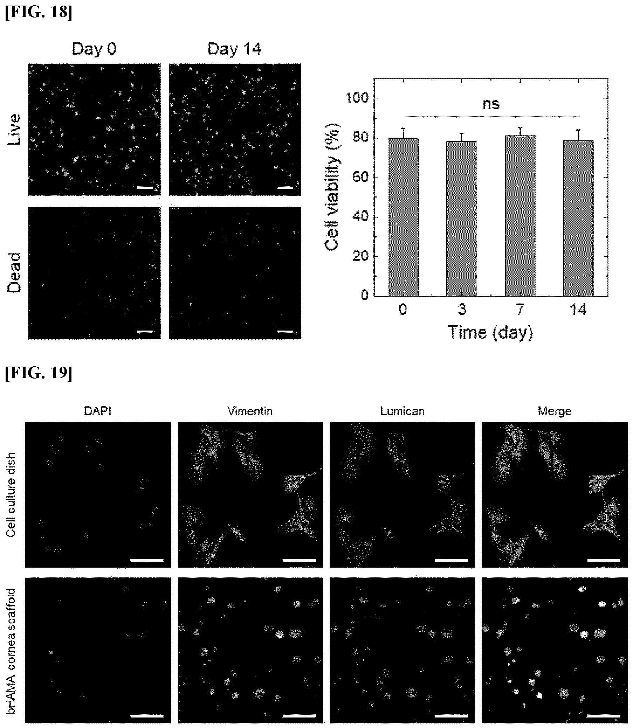

FIG. 18 shows results of a cell viability-time of Example 11 according to the present disclosure. The cell viability of the hydrogel of Example 11 was evaluated. The cell viability evaluation method was carried out in the same manner as Experimental Example 4. During the culture in medium for 14 days, corneal stromal cells in the hydrogel had cell damage in the course of the photo-curing. However, even after initial cell death, a favorable viability (about 80% or higher) was maintained.

FIG. 19 shows images identifying the characteristics of rabbit corneal stromal cells of Example 11 according to the present disclosure. The characteristics of the rabbit corneal stromal cells of Example 11 were investigated using immunohistochemical staining. The rabbit corneal stromal cells of Example 11 expressed vimentin and lumican and exhibited the same distinctive immunotypes as the rabbit corneal stromal cells that were cultured on the 2D surface. This showed that the phenotypes of rabbit corneal stromal cells did not change even after the DLP-based 3D bioprinting process.

While preferred embodiments of the present disclosure have been described above, those skilled in the art will appreciate that various modifications and changes can be made in the present disclosure within a scope without departing from the spirit and scope of the present disclosure set forth in the appended claims.

## Claims

1. A photoinitiator which has a chemical structure of the following Chemical Formula 1 and is water-soluble and activated by visible light to form free radicals: 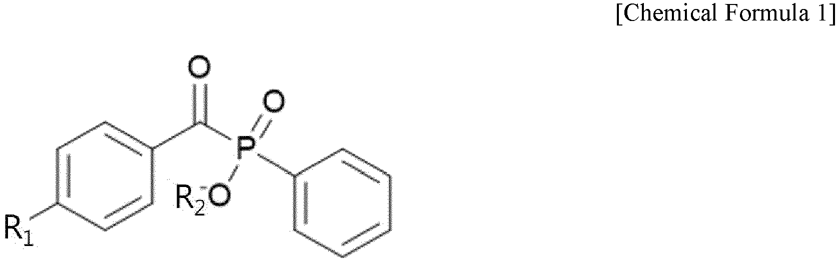 wherein, in the Chemical Formula 1, R₁ is H or F, and R₂ is Li⁺ or Mg²⁺.

2. The photoinitiator of claim 1, wherein a solubility of the photoinitiator in water at 25°C is 160 to 200 mM.

3. The photoinitiator of claim 1 or 2, wherein a molar extinction coefficient of the photoinitiator is 40 to 80 M⁻¹cm⁻¹.

4. A bioink composition, comprising:
distilled water;
a methacrylated hyaluronic acid (HAMA) compound dissolved in the distilled water; and
a photoinitiator which is dissolved in the distilled water and has a chemical structure of the following Chemical Formula 1: 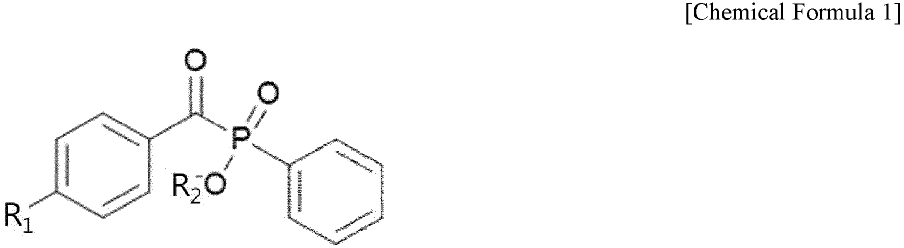
wherein, in the Chemical Formula 1, R₁ is H or F, and R₂ is Li⁺ or Mg²⁺.

5. The bioink composition of claim 4, wherein the methacrylated hyaluronic acid compound comprises:
a first methacrylated hyaluronic acid compound with a molecular weight of 1 to 20 kDa; and
a second methacrylated hyaluronic acid compound with a molecular weight of 50 to 2000 kDa.

6. The bioink composition of claim 5, wherein the methacrylated hyaluronic acid compound comprises the first methacrylated hyaluronic acid compound and the second methacrylated hyaluronic acid compound in a molar ratio of 2 to 7:8 to 3.

7. The bioink composition of any of claims 4 to 6, wherein the bioink composition comprises 5 to 15 mass% of the methacrylated hyaluronic acid compound and 0.02 to 5 % of the photoinitiator.

8. The bioink composition of any of claims 4 to 7, wherein a viscosity of the bioink composition is 30 to 70 mPa·s.

9. The bioink composition of any of claims 4 to 8, wherein the bioink composition further comprises:
a photoabsorber, optionally wherein the photoabsorber comprises tartrazine;
a cell, optionally comprising a corneal stromal cell; and/or
collagen.

10. The bioink composition of any of claims 4 to 9, wherein the bioink composition is for corneal formation.

11. A method of manufacturing a hydrogel, the method comprising:
dissolving a methacrylated hyaluronic acid (HAMA) compound and a photoinitiator with a chemical structure of the following Chemical Formula 1 in distilled water to prepare a bioink composition;
printing the bioink composition while irradiating visible light to crosslink the methacrylated hyaluronic acid (HAMA) compound: 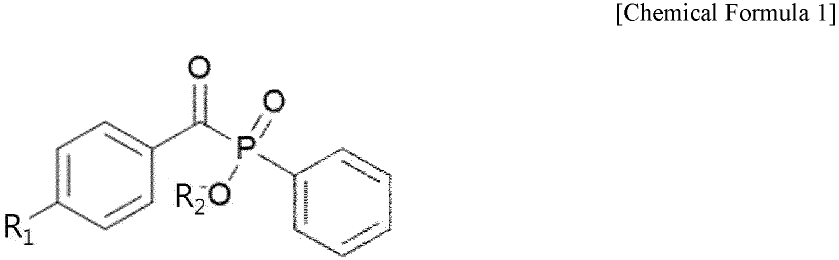
wherein, in the Chemical Formula 1, R₁ is H or F, and R₂ is Li⁺ or Mg²⁺.

12. The method of claim 11, wherein the methacrylated hyaluronic acid (HAMA) compound has a single or a plurality of molecular weights within a range of 1 to 2000 kDa, and
a toughness of the hydrogel is 10 to 40 kJ/m³.

13. The method of claim 11 or 12, wherein a thickness of the hydrogel is 300 to 700 µm.

14. The method of any of claims 11 to 13, wherein a transmittance of the hydrogel in light with a wavelength of 450 to 600 nm is 70 to 98%.

15. The method of any of claims 11 to 14, wherein:
(A) the tensile strength of the hydrogel is 150 to 250 kPa;
(B) the elongation of the hydrogel is 20 to 55%; and/or
(C) the toughness of the hydrogel is 10 to 40 kJ/m³.
